# EUROPEAN PATENT SPECIFICATION

(11) **EP 1 144 543 B1**
(45) Date of publication and mention of the grant of the patent: **17.03.2004**
(21) Application number: 99973057.5
(22) Date of filing: 01.12.1999
(51) Int. Cl.: C09K 11/06, H05B 33/14, C07D 215/30

(54) **ELECTROLUMINESCENT QUINOLATES**
ELEKTROLUMINESZENTE CHINOLATE
QUINOLATES ELECTROLUMINESCENTS

(30) Priority: 02.12.1998 GB 9826406
(43) Date of publication of application: 17.10.2001
(73) Proprietor: SOUTH BANK UNIVERSITY ENTERPRISES LTD., London SE1 0AA (GB)
(72) Inventor: KATHIRGAMANATHAN, Poopathy, North Harrow Middlesex HA2 7AP (GB)
(74) Representative: Lucas, Brian Ronald
(86) International application number: PCT/GB1999/004024
(87) International publication number: WO 2000/032717

(56) References cited:
- EP-A- 0 569 827
- EP-A- 0 936 844
- WO-A-98/02018
- US-A- 5 755 999
- HENSEN, KARL ET AL: "Preparation of N- or O-chloromethylsilyl derivatives of amines 1,2,3,4-tetrahydro-1,10-phenanthroline and 8-hydroxyquinoline" J. ORGANOMET. CHEM. (1981), 209(1), 17-23 , XP002130687
- PATENT ABSTRACTS OF JAPAN vol. 018, no. 462 (C-1243), 29 August 1994 (1994-08-29) & JP 06 145146 A (CHISSO CORP), 24 May 1994 (1994-05-24)

## Description

The present invention relates to novel photoluminescent and electroluminescent materials, to processes for their preparation and to electroluminescent devices containing them.

The obtaining of blue light in an electroluminescent material is required to enable the complete range of colours to be obtained in devices incorporating such materials

EP-A-0569827 discloses an electroluminescent device in which adhesion between the light-emitting layer and the cathode can be improved by providing an intermediate adhesive layer that includes a metal complex of 8-hydroxyquinoline and at least one organic compound, for example pentacene, tetracene, rubrene, tetrabenzoperylene, benzoperylene, coronene, perylene, benzotetracene, dobenzoanthracene and quinacridone. The light emitting layer is required to have an energy gap greater than that of the 8-hydroxyquinoline or metal complex thereof contained in the adhesive layer and is a compound that emits blue or blueish green light in CIE chromacity coordinates. In the examples such compounds include 4,4'-bis(2,2-diphenylvinyl)biphenyl and 1,1,4,4-tetraphenyl-1,3-butadiene. Complexes of 8-hydroxyquinoline mentioned for incorporation into the adhesive layer include tris(8-quinolinol)aluminum, bis(8-quinolinol)magnesium, bis(benzo-8-quinolinol)zinc, bis(2-methyl-8-quinolato)aluminumoxide, tris(8-quinolinol)indium, tris(5-methyl-8-quinolinol)aluminum, 8-quinolinol lithium, tris(5-chloro-8-quinolinol)gallium, bis(5-chloro-8-quinolinol)calcium, tris(5,7-dichloro-8-quinolinol)aluminum, tris(5,7-dibromo-8-hydroxyquinolinol)aluminum, bis(8-quinolinol)beryllium, bis(2-methyl-8-quinolinol)beryllium, bis(8-quinolinol)zinc, bis(2-methyl-8-quinolinol)zinc, bis(8-quinolinol)tin, and tris(7-propyl-8-quinolinol)aluminum. The thickness of the adhesive layer is less than that of the light-emitting layer in order to preserve the blue colour of the emitted light.

US-A-5755999 discloses blue-emitting electroluminescent devices based on organometallic complexes having *o*-(N-aryl-2-benzimidazolyl) phenol ligands. Examples use electroluminescent layers based on bis-[2-(2'-hydroxyphenyl)-1-phenyl-1H-benzimidazolate] beryllium (1931 CIE colour coordinates X=0.158, Y= 0.168), tris-[2-(2'-hydroxyphenyl)-1-phenyl-1H-benzimidazolate] aluminium (1931 CIE colour coordinates X=0.153, Y= 0.125), and tris-[2-(2'-hydroxyphenyl)-1-phenyl-1H-benzimidazolate] beryllium (1931 CIE colour coordinates X=0.154, Y= 0.151).

Aluminium quinolate is a known photoluminescent and electroluminescent material and emits light in the red area of the spectrum. In order to obtain light of a different wavelength dopants and/or dyes have been added to the aluminium quinolate. Structures have also been made with a layer containing dyes in contact with the aluminium quinolate layer but aluminium quinolate and structures based on aluminium quinolate have a relatively low efficiency.

In an article by Takeo Wakimoto et al in Applied Surface Science 113/114 (1997) 698-704 electroluminscent cells are disclosed in which aluminium quinolate is used as the emitter and which is doped by quinacrodine derivatives which are fluorescent dyes to change the colour of the emitted light.

WO 9802018 discloses an electroluminescent device comprising a substrate, an anode, an electroluminescent element and a cathode, wherein at least one of the two electrodes is transparent in the visible spectral range. The electroluminescent electrode can contain, in order a hole-injecting zone, hole-transporting zone, electroluminescent zone, electron-transporting zone and/or an electron-injecting zone. Characterizing features of the invention are that:
(a) the hole-injecting and/or hole-transporting zone is an optionally substituted tris-1,3,5-(aminophenyl)benzene compound A) or a mixture thereof; and
(b) the electroluminescent element optionally contains a further functionalised compound selected from among the hole-transporting materials, a luminescent material B and optionally electron-transporting materials. The electroluminescent component B may be selected from 8-hydroxyquinoline complexes of Al, Mg, In, Li, Ca, Na or aluminium tris(5-methyloxine) and gallium tris(5-chloroquinoline) or rare earth metal complexes. Examples employ aluminium tris(8-hydroxyquinolate) which gives a green or blue electroluminescence.

EP-A-0936844 is concerned with organic electroluminescent devices and disclosed in an example a green-emitting electroluminescent device having an electroluminescent layer of tris(8-hydroxyquinolato) aluminium of thickness 700Å and an electron injection layer of lithium 8-hydroxyquinolate deposited at a pressure of 10⁻⁶ Torr (1.33 x 10-⁴ Pa) and having a thickness of about 10Å.

JP 6145146 discloses new compounds useful as luminous materials or as electroluminescent elements and having the formula: 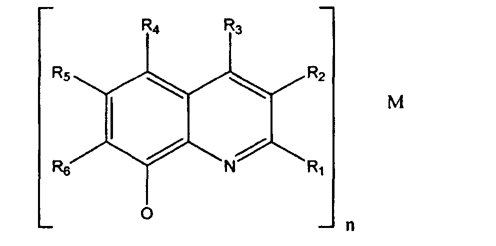 wherein R₁ to R₆ represent H, F or CN and at least one of them is F or CN, M represents a metal and n is 1-4. Compounds represented by the above formula may be made by dissolving, e.g. 5-cyano-8-quinolatolithium in a solvent, e.g. acetic acid, methanol, ethanol or propanol, adding the resultant solution to an aqueous solution containing a desired metal, regulating the pH, depositing a crystal and separating the deposited crystal by filtration. The resulting compounds are stated to be useful as components of EL elements and to be capable of emitting light of a high brightness at a low voltage. Various luminous colors can be obtained by changing the kind, position or number of the substituent group or the kind of the metal.

Electroluminescent devices can be made as described in an article by K. Nagayama et al in the Jpn. Journal of Applied Physics vol. 36 pps. 1555-1557.

A paper by Hensen *et al*., *J. Organometallic Camenistry*, **209** (1981) 17-23 discloses *inter alia* the synthesis of lithium 8-hydroxyquinolate by reacting 8-hydroxyquinoline in ether with butyl lithium in *n*-hexane. The lithium 8-hydroxyquinolate is then further reacted with (CH₃)ₙSiCl₄₋ₙ to produce 0-chloromethylsilyl derivatives of quinoline.

We have now obtained a lithium quinolate that can provide a blue electroluminescence and is as defined in claim 1 of the accompanying claims.

It was surprising that lithium quinolate could exhibit photoluminesce and electroluminesce in the blue spectrum.

The invention further provides a method of making a lithium quinolate having blue electroluminescence, which is as defined in claim 2 of the accompanying claims. Alkali metal alkyls are difficult compounds to handle practically as they are highly reactive and can catch fire spontaneously in air. For this reason they would not normally be chosen as reactants.

In the above method the lithium alkyl or alkoxide is preferably reacted in the liquid phase with the 8-hydroxyquinoline. The lithium compound can be dissolved in an inert solvent added to the 8-hydroxyquinoline. The lithium quinolate can be separated by evaporation or when a film of the lithium quinolate is required, by deposition onto a suitable substrate. The preferred alkyls are ethyl, propyl and butyl with n-butyl being particularly preferred. With lithium alkoxides the preferred alkoxides are ethoxide, propoxides and butoxides.

Lithium quinolate is synthesised by the reaction, in acetonitrile, of 8-hydroxyquinoline with a lithium alkyl e. g. n-butyl lithium. The lithium quinolate is an off white or white solid at room temperature.

A further aspect of the invention is the provision of a structure which incorporates a layer of lithium quinolate and a means to pass an electric current through the lithium quinolate layer. Thus the invention may provide an electroluminescent device having the features set out in claim 5 of the accompanying claims.

As well as the lithium salt of 8-hydroxyquinoline, the term quinolate in this specification includes salts of substituted 8-hydroxyquinoline 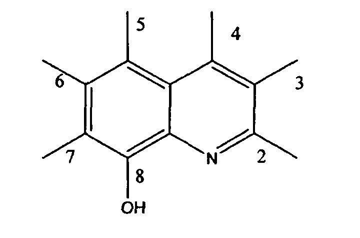 where the substituents are the same or different in the 2,3,4,5,6 and 7 positions and are selected from alky, alkoxy, aryl, aryloxy, sulphonic acids, esters, carboxylic acids, amino and amido groups or are aromatic, polycyclic or heterocyclic groups.

An electroluminescent device comprises a conductive substrate which acts as the anode, a layer of the electroluminescent material and a metal contact connected to the electroluminescent layer which acts as the cathode. When a current is passed through the electroluminescent layer, the layer emits light.

Preferably the electroluminescent devices of the invention comprise a transparent substrate, which is a conductive glass or plastic material which acts as the anode, preferred substrates are conductive glasses such as indium tin oxide coated glass, but any glass which is conductive or has a conductive layer can be used. Conductive polymers and conductive polymer coated glass or plastics materials can also be used as the substrate. The lithium quinolate can be deposited on the substrate directly by evaporation from a solution in an organic solvent. Any solvent which dissolves the lithium quinolate can be used e. g. acetonitrile.

A preferred method of forming a metal quinolate film e. g. useful in electroluminescent devices comprises forming the metal quinolate in situ by sequential dip coating the susbtrate with the film e. g. the substrate is dipped or otherwise coated with a solution of the metal alkyl or alkoxide to form a film on the surface and then dipped or otherwise coated with 8-hydroxyquinoline or substituted 8-hydroxyquinoline and the metal quinolate film is formed on the substrate surface.

For example to form a film of lithium quinolate the film or layer of lithium quinolate is deposited by in situ dip coating i. e. the substrate, such as a glass slide, is dipped into or otherwise contacted with a solution of an alkyl lithium e. g. n-butyl lithium and then immersed in or contacted with a solution of hydroxyquinoline, a layer of lithium quinolate is then formed on the substrate.

Alternatively the material can be deposited by spin coating or by vacuum deposition from the solid state e. g. by sputtering or any other conventional method can be used.

To form an electroluminescent device incorporating lithium quinolate as the emissive layer there can be a hole transporting layer deposited on the transparent substrate and the lithium quinolate is deposited on the hole transporting layer. The hole transporting layer serves to transport; holes and to block the electrons, thus preventing electrons from moving into the electrode without recombining with holes.

The recombination of carriers therefore mainly takes place in the emitter layer.

Hole transporting layers are used in polymer electroluminescent devices and any of the known hole transporting materials in film form can be used. The hole transporting layer can be made of a film of an aromatic amine complex such as poly (vinylcarbazole), N, N'-diphenyl-N, N'-bis (3-methylphenyl)-I, I'-biphenyl-4,4'diamine (TPD), polyaniline etc.

Optionally dyes such as fluorescent laser dyes, luminescent laser dyes can be included to modify the colour spectrum of the emitted light.

The lithium quinolate can be mixed with a polymeric material such as a polyolefin e.g. polyethylene, polypropylene etc. and preferably polystyrene. Preferred amounts of active material in the mixture is from 95% to 5% by weight of active material and more preferably 25 to 20% by weight.

The hole transporting material can optionally be mixed with the lithium quinolate in a ratio of 5-95% of the lithium quinolate to 95 to 5% of the hole transporting compound. In another embodiment of the invention there is a layer of an electron injecting material between the cathode and the lithium quinolate layer. This electron injecting layer is preferably a metal complex such as a different metal quinolate e. g. an aluminium quinolate which will transport electrons when an electric current is passed through it. Alternatively the electron injecting material can be mixed with the lithium quinolate and co-deposited with it.

In another embodiment of the invention there is a layer of an electron transporting material between the cathode and the lithium quinolate layer, this electron transporting layer is preferably a metal complex such as a metal quinolate e. g. an aluminium quinolate which will transport electrons when an electric current is passed through it. Alternatively the electron transporting material can be mixed with the lithium quinolate and co-deposited with it.

Optionally dyes such as fluorescent laser dyes, luminescent laser dyes can be included to modify the colour spectrum of the emitted light and also enhance the photoluminescent and electroluminescent efficiencies.

In a preferred structure there is a substrate formed of a transparent conductive material which is the anode on which is successively deposited a hole transportation layer, the lithium quinolate layer and an electron transporting layer which is connected to the anode. The anode can be any low work function metal e. g. aluminium, calcium, lithium, silver/magnesium alloys etc.

The invention is further described with reference to the examples in which Examples 3-7 are for comparative purposes

### Example 1 Lithium 8-hydroxyquinolate Li (C₉H₆ON)

2.32g (0.016 mole) of 8-hydroxyquinoline was dissolved in acetonitrile and 10ml of 1.6M n-butyl lithium (0.016 mole) was added. The solution was stirred at room temperature for one hour and an off white precipitate filtered off. The precipitate was washed with water followed by acetonitrile and dried in vacuo. The solid was shown to be lithium quinolate.

### Example 2 1 Lithium 8-hydroxyquinolate Li (C₉H₆ON)

A glass slide (Spectrosil UV grade) was dipped into a solution of n-butyl lithium in acetonitrile for four seconds and then in immersed in a solution of 8hydroxyquinoline for four seconds. A thin layer of lithium quinolate was easily seen on the glass.

### Example 3 Magnesium 8-hydroxyquinolate Mg(C₉H₆ON)₂

8-Hydroxyquinoline (5.0-g; 0.0345 mole) was dissolved in 2N acetic acid (150 ml) by heating at 70-80 C. Magnesium sulphate (2.5 g; 0.020 mole) was dissolved in water (100 ml) heated to 60 C and basified with ammonia. Oxine solution was added to the mechanically stirred, basified magnesium sulphate solution at 60 C and excess ammonia added until the pH of the solution was 9.5. The yellow precipitate was digested at 60 C for a further 10 minutes, cooled and filtered under suction, washed with dilute ammonia and dried in vacuo at 100 C for several hours. Yield 5.06. g

### Example 4 Zinc 8-hydroxyquinolate Zn(C₉H₆ON)₂

The above procedure was employed using 8-hydroxyquinoline (5.0 g; 0.0345 mole) and zinc chloride (2.8 g; 0.020 mole). The yellow precipitate was filtered, washed with dilute ammonia and dried in vacuo at 75°C for 6 hours. Yield 6.48 g

### Example 5 Calcium 8-hydroxyquinolate Ca(C₉H₆ON)₂

Using similar procedure with 8-hydroxyquinoline (5.0 g; 0.0345 mole) and calcium chloride (3.8 g; 0.034 mole), calcium 8-hydroxyquinolate was obtained as a yellow powder 5.60 g yield.

### Example 6 Sodium 8-hydroxyquinolate Na(C₉H₆ON)

8-Hydroxyquinoline (5.0 g; 0.0345 mole) was dissolved in 2 % sodium hydroxide solution (100 ml) and heated to 60°C. The solution was stirred at this temperature for 30 minutes and the homogeneous solution was cooled to room temperature. No solid was separated out. Therefore the solution was concentrated in a rotary evaporator and the concentrated solution was cooled to give a pale yellow solid. The solid was filtered under suction washed with small amounts of sodium hydroxide solution and dried in vacuo at 80°C for several hours. The sodium 8-hydroxyquninolate is soluble in water. Yield 3.6 g.

### Example 7 Potassium 8-hydroxyquinolate K(C₉H₆ON)

Potassium 8-hydroxyquinolate was also made from 8-hydroxyquinoline (2.0 g; 0.0138 mole) in dry tetrahydrofuran (50 ml) and potassium tert-butoxide (2.32 g; 0.021 mole). The solution was heated to become homogeneous and cooled to room temperature to give a yellow solid yield 2.2 g.

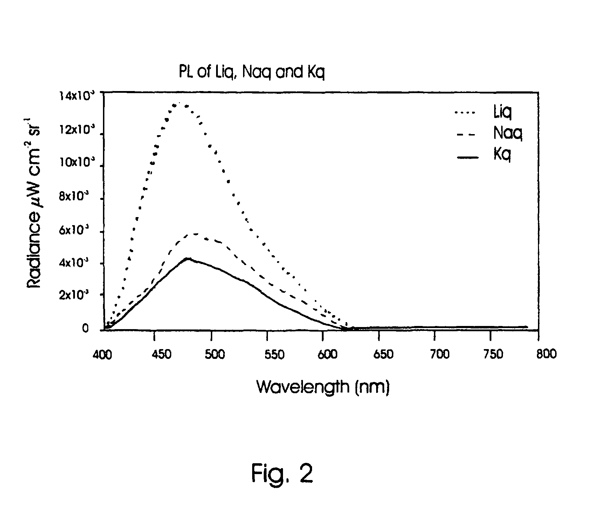

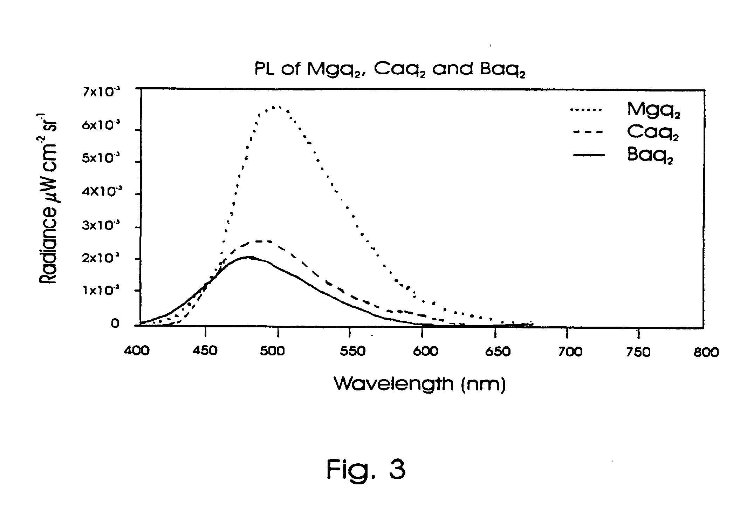

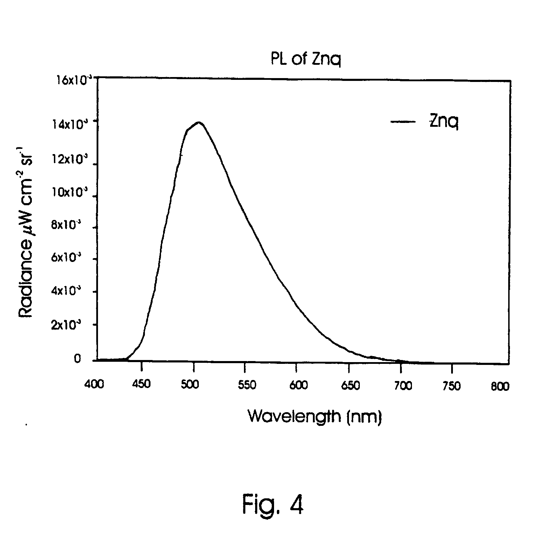

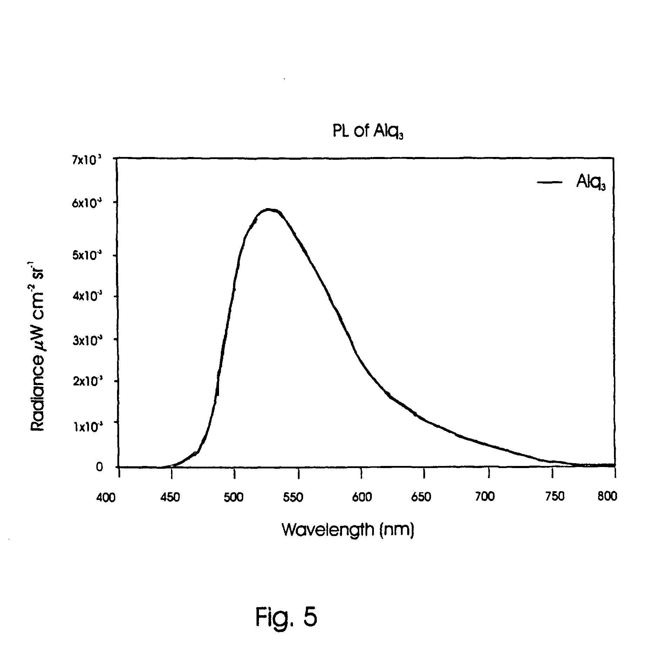

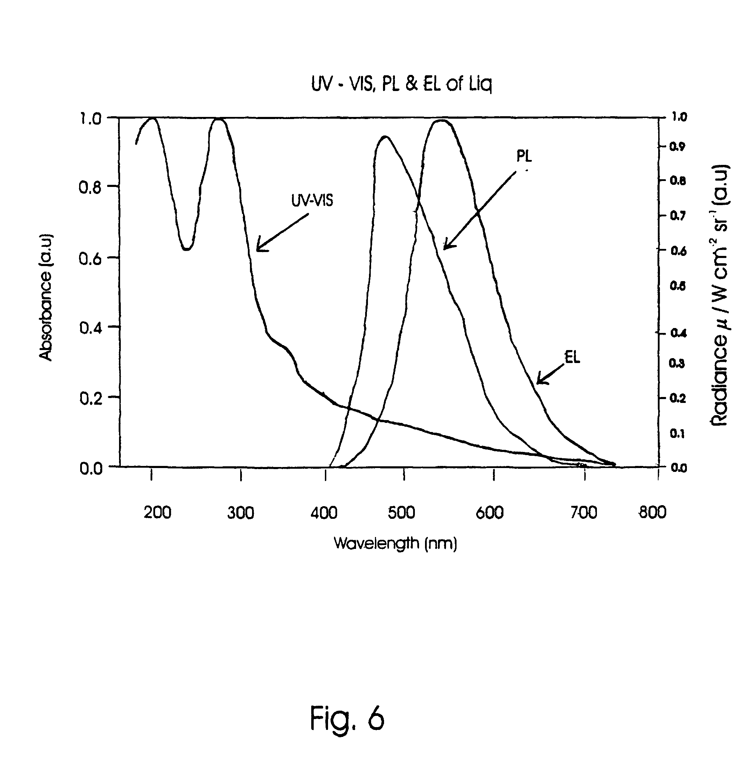

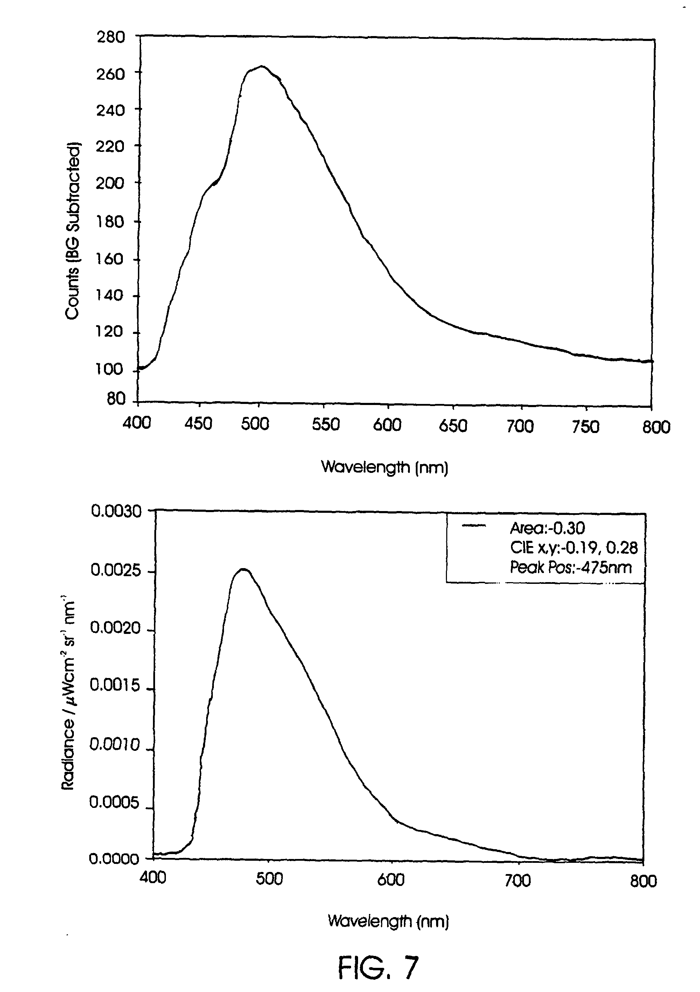

The photoluminescent efficiency and maximum wavelength of the PL emission of the lithium quinolate was measured and compared with other metal quinolates and the results shown in Table 1. Photoluminescence was excited using 325mn line of Liconix 4207 NB, He/Cd laser. The laser power incident at the sample (0.3mWcm-2) was measured by a Liconix 55PM laser power meter. The radiance calibration was carried out using Bentham radiance standard (Bentham SRS8, Lamp current 4,000A), calibrated by National Physical laboratories, England. The PL studies were carried out on samples or films. The Spectra are attached as Figs. 2 to 7.

**Table 1**

| | | | Absolute Photoluminescent Efficiency % |
|---|---|---|---|
| **Complex** | **CIE x,y** | **λ**_{**max**} **(PL)/nm** | **ηPL** |
| Liq | 0.17, 0.23 | 465 | 48 |
| Naq | 0.19, 0.31 | 484 | 32 |
| Kq | 0.19,0.33 | 485 | 36 |
| Baq₂ | 0.16, 0.29 | 479 | 7 |
| Caq₂ | 0.21,0.37 | 482 | 24 |
| Mgq₂ | 0.22, 0.46 | 500 | 43 |
| Znq₂ | 0.26, 0.51 | 518 | |
| Alq₃ | 0.32, 0.56 | 522 | 27 |

### Example 8

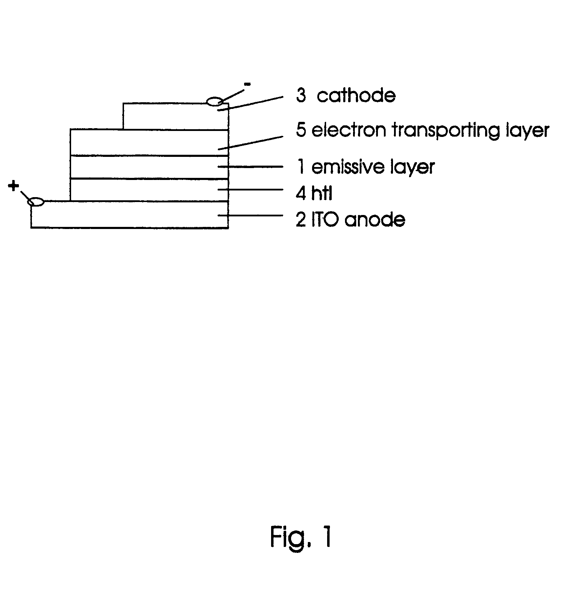

An electroluminescent device of structure shown in fig. 1 was fabricated using aluminium quinolate and lithium quinolate as the electroluminescent layer and the electroluminescent properties measured. Referring to fig. 1 (2) is an ITO layer, (4) is a TPD layer (hole transporting layer) (60nm), (1) is the lithium quinolate layer (5) is an aluminium quinolate layer and (3) is aluminium (900nm).

### 1. Device Fabrication

An ITO coated glass piece (1 x 1cm² cut from large sheets purchased from Balzers, Switzerland) had a portion etched out with concentrated hydrochloric acid to remove the ITO and was cleaned and placed on a spin coater (CPS 10 BM, Semitec, Germany) and spun at 2000 rpm for 30 seconds, during which time the solution of the electroluminescent compound was dropped onto it dropwise by a pipette.

Alternatively the electroluminescent compound was vacuum evaporated onto the ITO coated glass piece by placing the substrate in a vacuum coater and evaporating the electroluminescent compound at 10⁻⁵ to 10⁻⁶ torr onto the substrate.

The organic coating on the portion which had been etched with the concentrated hydrochloric acid was wiped with a cotton bud.

The coated electrodes were stored in a vacuum desiccator over calcium sulphate until they were loaded into a vacuum coater (Edwards, 10⁻⁶ torr) and aluminium top contacts made. The active area of the LED's was 0.08 cm² by 0.I cm² the devices were then kept in a vacuum desiccator until the electroluminescence studies were performed.

The ITO electrode was always connected to the positive terminal. The current vs. voltage studies were carried out on a computer controlled Keithly 2400 source meter.

Electroluminescence spectra were recorded by means of a computer controlled charge coupled device on Insta Spec photodiode array system model 77112 (Oriel Co. Surrey, England)

The spectra are shown in the drawings.

In the spectra:-
Fig. 2 shows the PL of lithium 8-hydroxyquinolate of Example 1 and the quinolates of Examples 6 and 7.
Fig. 3 shows the PL of quinolates of Examples 3 and 5 and that of barium quinolate made by the same method.
Fig. 4 shows the PL of zinc quinolate of Example 4
Fig. 5 shows the PL of commercially available aluminium quinolate and
Fig. 6 shows the UV-VIS, PL and EL of lithium quinolate and
Fig. 7 shows the spectra of lithium quinolate of Example 2

## Claims

1. A lithium quinolate or substituted quinolate that provides a blue electroluminescence and is obtainable by the reaction of lithium alkyl or alkoxide in a solvent comprising acetonitrile with 8-hydroxyquinoline which may be unsubstituted or may have substituents selected from alkyl, alkoxy, aryl, aryloxy, sulphonic acid, ester, carboxylic acid, amino or amido groups or aromatic, polycyclic or heterocyclic groups.

2. A method of making a lithium quinolate or substituted quinolate that provides a blue electroluminescence, which comprises reacting a lithium alkyl or alkoxide in a solvent comprising acetonitrile with 8-hydroxyquinoline which may be unsubstituted or may have substituents which are selected from alkyl, alkoxy, aryl, aryloxy, sulphonic acid, ester, carboxylic acid, amino or amido groups or aromatic polycyclic or heterocyclic groups.

3. The method of claim 2, wherein ethyl, propyl or butyl lithium is reacted with the 8-hydroxyqinoline or substituted 8-hydroxyquinoline.

4. The method of claim 2, wherein n-butyl lithium is reacted with the 8-hydroxyquinoline or substituted 8-hydroxyquinoline.

5. An electroluminescent device comprising sequentially a first electrode, a layer of a blue-emissive electroluminescent material and a second electrode **characterised in that** the blue-emissive electroluminescent material is a lithium quinolate or substituted quinolate obtainable by the reaction of lithium alkyl or alkoxidc in a solvent comprising acetonitrile with 8-hydroxyquinoline which may be unsubstituted or may have substituents which are selected from alkyl, alkoxy, aryl, aryloxy, sulphonic acid, ester, carboxylic acid, amino or amido groups or aromatic, polycyclic or heterocyclic groups.

6. The device of claim 5 wherein there is a layer of a hole transporting material on the conductive substrate and the lithium quinolate is on the layer of hole transporting material.

7. The device of claim 6, wherein the hole transporting material is a polyamine.

8. The device of claim 7, wherein the hole transporting layer is poly(vinylcarbazole) or N,N'-diphenyl-N,N'-bis (3-methylphenyl)-1,1'-biphenyl -4,4'- 10 diamine (TPD).

9. The device of claim 7, wherein the hole transporting layer is of a polyaniline.

10. The device of any of claims 5-9, wherein the hole transporting material is mixed with lithium quinolate in a ratio of 5-95% by weight of the lithium quinolate to 95-5% by weight of the hole transporting material.

11. The device of any of claims 5-10, wherein the lithium quinolate is mixed with a polyolefin and the amount of lithium quinolate in the mixture is from 95% to 5% by weight of the mixture.

12. The device of any of claims 5-11, wherein there is a layer of an electron injecting material between the cathode and the lithium quinolate layer.

13. The device of any of claims 5 to 12, wherein there is an electron injecting material mixed with the lithium quinolate

14. The device of claim 12 or 13, wherein the electron injecting material is aluminium quinolate which will transport electrons when an electric current is passed through it.

15. The device of any of claims 5 -14 wherein the anode is a conductive glass.

16. An electroluminescent device which comprises sequentially a conductive substrate which acts as the anode, a layer of a hole transporting material, a layer of lithium quinolate, a layer of an electron injecting material and a metal which acts as the cathode **characterised in that** the lithium quinolate is a blue emissive lithium quinolate made by the reaction of a lithium alkyl or alkoxide with 8-hydroxyquinoline or substituted 8-hydroxyquinoline in a solution in a solvent which comprises acetonitrile.

17. A method of forming an electroluminescent device comprising sequentially a first electrode, a layer of an electroluminescent material and a second electrode which comprises depositing the electroluminescent material on the first electrode **characterised in that** the method comprises reacting a lithium alkyl or alkoxide with 8-hydroxyquinoline in a solvent comprising acetonitrile to form a blue emissive lithium quinolate and depositing the lithium quinolate formed on the first electrode.

## Patentansprüche

1. Lithiumchinolat oder substituiertes -chinolat, das eine blaue Elektrolumineszenz liefert und durch die Umsetzung eines Lithiumalkyls oder -alkoxids in einem Acetonitril umfassenden Lösungsmittel mit 8-Hydroxychinolin, das unsubstituiert sein kann oder Substituenten aufweisen kann, die aus Alkyl-, Alkoxy-, Aryl-, Aryloxy-, Sulfonsäure-, Ester-, Carbonsäure-, Amino- oder Amidogruppen oder aromatischen, polycyclischen oder heterocyclischen Gruppen ausgewählt sind, erhalten werden kann.

2. Verfahren zur Herstellung eines Lithiumchinolats oder substituierten -chinolats, das eine blaue Elektrolumineszenz liefert, das die Umsetzung eines Lithiumalkyls oder -alkoxids in einem Acetonitril umfassenden Lösungsmittel mit 8-Hydroxychinolin, das unsubstituiert sein kann oder Substituenten aufweisen kann, die aus Alkyl-, Alkoxy-, Aryl-, Aryloxy-, Sulfonsäure-, Ester-, Carbonsäure-, Amino- oder Amidogruppen oder aromatischen, polycyclischen oder heterocyclischen Gruppen ausgewählt sind, umfaßt.

3. Verfahren nach Anspruch 2, wobei Ethyl-, Propyl- oder Butyllithium mit dem 8-Hydroxychinolin oder dem substituierten 8-Hydroxychinolin umgesetzt wird.

4. Verfahren nach Anspruch 2, wobei n-Butyllithium mit dem 8-Hydroxychinolin oder dem substituierten 8-Hydroxychinolin umgesetzt wird.

5. Elektrolumineszierende Vorrichtung, die nacheinander eine erste Elektrode, eine Schicht eines Blau emittierenden, elektrolumineszierenden Materials und eine zweite Elektrode umfaßt, **dadurch gekennzeichnet, daß** das Blau emittierende, elektrolumineszierende Material ein Lithiumchinolat oder substituiertes -chinolat ist, das durch die Umsetzung eines Lithiumalkyls oder -alkoxids in einem Acetonitril umfassenden Lösungsmittel mit 8-Hydroxychinolin, das unsubstituiert sein kann oder Substituenten aufweisen kann, die aus Alkyl-, Alkoxy-, Aryl-, Aryloxy-, Sulfonsäure-, Ester-, Carbonsäure-, Amino- oder Amidogruppen oder aromatischen, polycyclischen oder heterocyclischen Gruppen ausgewählt sind, erhalten werden kann.

6. Vorrichtung nach Anspruch 5, wobei eine Schicht eines Elektronenlücken transportierenden Materials auf dem leitfähigen Substrat vorliegt und sich das Lithiumchinolat auf der Schicht des Elektronenlücken transportierenden Materials befindet.

7. Vorrichtung nach Anspruch 6, wobei das Elektronenlücken transportierende Material ein Polyamin ist.

8. Vorrichtung nach Anspruch 7, wobei das Elektronenlücken transportierende Material Poly(vinylcarbazol) oder N,N'-Diphenyl-N,N'-bis(3-methylphenyl)-1,1'-biphenyl-4,4'-10-diamin (TPD) ist.

9. Vorrichtung nach Anspruch 7, wobei das Elektronenlücken transportierende Material ein Polyanilin ist.

10. Vorrichtung nach einem der Ansprüche 5 bis 9, wobei das Elektronenlücken transportierende Material mit Lithiumchinolat in einem Verhältnis von 5 bis 95 Gew.-% Lithiumchinolat zu 95 bis 5 Gew.-% Elektronenlücken transportierendes Material gemischt ist.

11. Vorrichtung nach einem der Ansprüche 5 bis 10, wobei das Lithiumchinolat mit einem Polyolefin gemischt ist und die Lithiumchinolatmenge im Gemisch 95 bis 5 Gew.-% des Gemisches beträgt.

12. Vorrichtung nach einem der Ansprüche 5 bis 11, wobei zwischen der Kathode und der Lithiumchinolatschicht eine Schicht eines Elektronen injizierenden Materials vorliegt.

13. Vorrichtung nach einem der Ansprüche 5 bis 12, wobei ein Elektronen injizierendes Material mit der Lithiumchinolatschicht gemischt ist.

14. Vorrichtung nach Anspruch 12 oder 13, wobei das Elektronen injizierende Material Aluminiumchinolat ist, das Elektronen transportiert, wenn elektrischer Strom hindurchfließt.

15. Vorrichtung nach einem der Ansprüche 5 bis 14, wobei die Anode ein leitfähiges Glas ist.

16. Elektrolumineszierende Vorrichtung, die nacheinander ein leitfähiges Substrat, das als Anode wirkt, eine Schicht eines Elektronenlücken transportierenden Materials, eine Lithiumchinolatschicht, eine Schicht eines Elektronen injizierenden Materials und ein Metall, das als Kathode wirkt, umfaßt, **dadurch gekennzeichnet, daß** das Lithiumchinolat ein Blau emittierendes Lithiumchinolat ist, das durch die Umsetzung eines Lithiumalkyls oder -alkoxids mit 8-Hydroxychinolin oder substituiertem 8-Hydroxychinolin in einer Lösung in einem Lösungsmittel, das Acetonitril umfaßt, hergestellt wurde.

17. Verfahren zur Herstellung einer elektrolumineszierenden Vorrichtung, die nacheinander eine erste Elektrode, eine Schicht eines elektrolumineszierenden Materials und eine zweite Elektrode umfaßt, das das Auftragen des elektrolumineszierenden Materials auf der ersten Elektrode umfaßt, **dadurch gekennzeichnet, daß** das Verfahren das Umsetzen eines Lithiumalkyls oder -alkoxids mit 8-Hydroxychinolin in einem Acetonitril umfassenden Lösungsmittel, wodurch ein Blau emittierendes Lithiumchinolat erzeugt wird, und das Auftragen des erzeugten Lithiumchinolats auf der ersten Elektrode umfaßt.

## Revendications

1. Quinoléate ou quinoléate substitué de lithium qui fournit une électroluminescence bleue et qui est susceptible d'être obtenue par la réaction d'un alkylure ou d'un alkoxyde de lithium dans un solvant comprenant l'acétonitrile avec la 8-hydroxyquinoléine qui peut être non substituée ou peut avoir des substituants choisis parmi les groupes alkyle, alcoxy, aryle, aryloxy, acide sulfonique, ester, acide carboxylique, amino ou amido ou les groupes aromatiques, polycycliques ou hétérocycliques.

2. Procédé pour fabriquer un quinoléate ou un quinoléate substitué de lithium qui fournit une électroluminescence bleue, qui comprend les étapes consistant à faire réagir un alkylure ou un alkoxyde de lithium dans un solvant comprenant l'acétonitrile avec la 8-hydroxyquinoléine qui peut être non substituée ou peut avoir des substituants qui sont choisis parmi les groupes alkyle, alcoxy, aryle, aryloxy, acide sulfonique, ester, acide carboxylique, amino ou amido ou les groupes aromatiques, polycycliques ou hétérocycliques.

3. Procédé selon la revendication 2, dans lequel l'éthyl-, le propyl- ou le butyl-lithium est mis à réagir avec la 8-hydroxyquinoléine ou la 8-hydroxyquinoléine substituée.

4. Procédé selon la revendication 2, dans lequel le n-butyl-lithium est mis à réagir avec la 8-hydroxyquinoléine ou la 8-hydroxyquinoléine substituée.

5. Dispositif d'électroluminescence comprenant séquentiellement une première électrode, une couche d'une matière électroluminescente émettrice de bleu et une deuxième électrode **caractérisé en ce que** la matière électroluminescente émettrice de bleu est un quinoléate ou un quinoléate substitué de lithium susceptible d'être obtenu par la réaction d'un alkyline ou d'un alkoxyde de lithium dans un solvant comprenant l'acétonitrile avec la 8-hydroxyquinoléine qui peut être non substitué ou peut avoir des substituants qui sont choisis parmi les groupes alkyle, alcoxy, aryle, aryloxy, acide sulfonique, ester, acide carboxylique, amino ou amido, ou les groupes aromatiques, polycycliques ou hétérocycliques.

6. Dispositif selon la revendication 5, dans lequel il existe une couche d'une matière transportant des trous sur le substrat conducteur et le quinoléate de lithium est sur la couche de matière transportant des trous.

7. Dispositif selon la revendication 6, dans lequel la matière transportant des trous est une polyamine.

8. Dispositif selon la revendication 7, dans lequel la couche transportant des trous est le poly(vinylcarbazole) ou la N,N'-diphényl-N,N'-bis(3-méthylphényl)-1,1'-biphényl-4,4'-10-diamine (TPD).

9. Dispositif selon la revendication 7, dans lequel la couche transportant des trous est faite d'une polyaniline.

10. Dispositif selon l'une quelconque des revendications 5 à 9, dans lequel la matière transportant des trous est mélangée avec un quinoléate de lithium dans un rapport de 5 à 95% en poids du quinoléate de lithium à 95 à 5% en poids d'une matière transportant des trous.

11. Dispositif selon l'une quelconque des revendications 5 à 10, dans lequel le quinoléate de lithium est mélangé avec une polyoléfine et la quantité de quinoléate de lithium dans le mélange va de 95% à 5% en poids du mélange.

12. Dispositif selon l'une quelconque des revendications 5 à 11, dans lequel il existe une couche d'une matière injectant des électrons entre la cathode et la couche de quinoléate de lithium.

13. Dispositif selon l'une quelconque des revendications 5 à 12, dans lequel il existe une matière injectant des électrons, mélangée avec le quinoléate de lithium.

14. Dispositif selon la revendication 12 ou 13, dans lequel la matière injectant des électrons est le quinoléate d'aluminium qui transportera les électrons quand un courant électrique le traverse.

15. Dispositif selon l'une quelconque des revendications 5 à 14, dans lequel l'anode est un verre conducteur.

16. Dispositif électroluminescent qui comprend séquentiellement un substrat conducteur qui agit en tant qu'anode, une couche d'une matière transportant des trous, une couche de quinoléate de lithium, une couche d'une matière injectant des électrons et un métal qui agit en tant que cathode, **caractérisé en ce que** le quinoléate de lithium est un quinoléate de lithium émetteur de bleu constitué par la réaction d'un alkylure ou alkoxyde de lithium avec la 8-hydroxyquinoléine ou la 8-hydroxyquinoléine substituée dans une solution dans un solvant qui comprend l'acétonitrile.

17. Procédé pour former un dispositif électroluminescent comprenant séquentiellement une première électrode, une couche de matière électroluminescente et une deuxième électrode qui comprend les étapes consistant à déposer la matière électroluminescente sur la première électrode, **caractérisé en ce que** le procédé comprend les étapes consistant à faire réagir un alkylure ou alkoxyde de lithium avec la 8-hydroxyquinoléine dans un solvant comprenant l'acétonitrile afin de former un quinoléate de lithium émetteur de bleu et à déposer le quinoléate de lithium formé sur la première électrode.
